# EUROPÄISCHE PATENTANMELDUNG

(11) **EP 0 887 363 A1**
(43) Veröffentlichungstag der Anmeldung: **30.12.1998**
(21) Anmeldenummer: 98108406.4
(22) Anmeldetag: 08.05.1998
(51) Int. Cl.: C08G 18/62, A61L 27/00, A61L 31/00

(54) **Blutverträgliches und bakterienabweisendes Copolymer**

(30) Priorität: 24.06.1997 DE 19726738
(71) Anmelder: HÜLS AKTIENGESELLSCHAFT, 45764 Marl (DE)
(72) Erfinder: Anders, Christine, Dr., 45721 Haltern (DE)

(57) **Zusammenfassung**

Die Erfindung betrifft ein Copolymer, enthaltend (a) mindestens ein sulfonsäure- oder sulfonatgruppenhaltiges Monomer, (b) mindestens ein carboxyl- oder carboxylatgruppenhaltiges Monomer und (c) mindestens ein Monomer mit einer NCO-reaktiven Gruppe. Das Copolymer kann bis zu 70 % Molprozent, bezogen auf die Summe der Monomeren (a), (b) und (c), Monomere mit anderer Funktionalität als derjenigen der genannten Monomeren oder ohne Funktionalität enthalten und kann durch Umsetzung mit einem Polyisocyanat kovalent auf hydrophilen Oberfläche von Polymersubstraten gebunden werden. Die Erfindung betrifft auch Erzeugnissen mit derart modifizierten Oberflächen und deren Verwendung für medizinische, hygienische, technische, nahrungsmittel- oder biotechnische Zwecke.

## Beschreibung

Die Erfindung betrifft ein blutverträgliches und bakterienabweisendes Copolymer, ein Verfahren zu dessen Herstellung, ein Verfahren zur Modifizierung von hydrophilen Oberflächen mit diesem Copolymer sowie Erzeugnisse mit derart modifizierten Oberflächen und deren Verwendung für verschiedene, in der Folge erläuterte Zwecke, insbesondere für medizinische Zwecke.

### 1. Stand der Technik

Die Ansiedelung und Vermehrung von Bakterien auf Oberflächen ist eine in der Regel unerwünschte Erscheinung, die häufig mit nachteiligen Folgen verbunden ist. So können in der Trinkwasser- und Getränketechnik Bakterienpopulationen zu einer gesundheitsgefährdenden Qualitätsminderung führen. Bakterien auf oder in Verpackungen bewirken häufig den Verderb von Lebensmitteln oder verursachen sogar Infektionen bei dem Verbraucher. In steril zu betreibenden biotechnischen Anlagen stellen systemfremde Bakterien ein erhebliches prozeßtechnisches Risiko dar. Solche Bakterien können mit Rohstoffen eingetragen werden oder bei mangelhafter Sterilisation in allen Anlageteilen zurückbleiben. Teile der Bakterienpopulation können sich durch Adhäsion dem normalen Flüssigkeitsaustausch beim Spülen und Reinigen entziehen und sich im System vermehren.

Weiterhin sind Bakterienansiedelungen in Wasseraufbereitungsanlagen (z.B. zur Entsalzung durch Membranen) oder auch in Behältern bekannt, die mit gelösten oder flüssigen unverdünnten organischen Substanzen gefüllt sind und für Bakterienpopulationen vorteilhafte Bedingungen aufweisen. Solche mikrobiellen Belegungen können in erheblichem Umfang zur Blockierung und/oder korrosiven Zerstörung der Anlage führen.

Besondere Bedeutung kommt dem Schutz vor Bakterienanhaftung und -ausbreitung in der Ernährung, der Pflege, hier insbesondere in der Altenpflege, und in der Medizin zu. Bei Massenbeköstigungen oder -ausschank existieren besonders dann erhebliche Risiken, wenn zur Vermeidung von Abfall von Einweggeschirr abgesehen wird und eine nur unzureichende Reinigung des Mehrweggeschirrs erfolgt. Die schädliche Ausbreitung von Bakterien in lebensmittelführenden Schläuchen und Rohren ist ebenso bekannt wie die Vermehrung in Lagerbehältern sowie in Textilien in feuchter und warmer Umgebung, z.B. in Bädern. Solche Einrichtungen sind bevorzugte Lebensräume für Bakterien, ebenso wie bestimmte Oberflächen in Bereichen mit hohem Publikumsverkehr. so z.B. in öffentlichen Verkehrsmitteln, Krankenhäusern, Telefonzellen, Schulen und insbesondere in öffentlichen Toiletten.

In der Alten- und Krankenpflege erfordern die häufig geminderten Abwehrkräfte der Betroffenen sorgfältige Maßnahmen gegen Infektionen, insbesondere auf Intensivstationen und in der häuslichen Pflege.

Besondere Sorgfalt bedarf die Verwendung medizinischer Gegenstände und Geräte bei medizinischen Untersuchungen. Behandlungen und Eingriffen, vor allem dann, wenn derartige Geräte oder Gegenstände mit lebendem Gewebe oder mit Körperflüssigkeiten in Kontakt kommen. Im Falle von Langzeit- oder Dauerkontakten, beispielsweise bei Implantaten, Kathetern, Stents, Herzklappen und Herzschrittmachern, können Bakterienkontaminationen zu einem lebensbedrohenden Risiko für den Patienten werden.

Es wurde bereits auf vielfältige Weise versucht, die Ansiedelung und Ausbreitung von Bakterien auf Oberflächen zu unterbinden. In J. Microbiol. Chemoth. **31** (1993), 261-271 beschreiben S.E. Tebbs und T.S.J.Elliott lackartige Beschichtungen mit quaternären Ammoniumsalzen als antimikrobiell wirkenden Komponenten. Es ist bekannt, daß diese Salze von Wasser, wäßrigen oder anderen polaren Medien sowie von Körperflüssigkeiten aus dem Beschichtungsmaterial herausgelöst werden und ihre Wirkung somit nur von kurzer Dauer ist. Dies gilt gleichermaßen für die Einarbeitung von Silbersalzen in Beschichtungen, so beschrieben in WO 92/18098.

T. Ouchi und Y. Ohya beschreiben in Progr.Polym.Sci. **20** (1995), 211 ff., die Immobilisierung von bakteriziden Wirkstoffen auf Polymeroberflächen durch kovalente Bindung oder ionische Wechselwirkungen. Häufig sind in solchen Fällen die keimtötenden Wirkungen gegenüber dem reinen Wirkstoff deutlich reduziert. Heteropolare Bindungen erweisen sich oft als nicht hinreichend stabil. Darüber hinaus führt die Keimabtötung in der Regel zu unerwünschten Ablagerungen auf den Oberflächen, die die weitere bakterizide Wirkung maskieren und die Grundlage für eine nachfolgende Bakterienbesiedelung bilden.

W.Kohnen et al. berichten in ZBl.Bakt.Suppl. 26, Gustav Fischer Verlag, Stuttgart-Jena-New York, 1994, Seiten 408 bis 410, daß die Adhäsion von Staphylococcus epidermidis auf einem Polyurethanfilm vermindert wird, wenn der Film durch eine Glimmentladung in Gegenwart von Sauerstoff vorbehandelt und dann mit Acrylsäure gepfropft wird.

Bei Gegenständen zur Verwendung für medizinische Zwecke, d.h. für Untersuchungen, Behandlungen und Eingriffe, wie zuvor beschrieben, spielen nicht nur die bakterienabweisenden Eigenschaften eine Rolle, vielmehr kommt es auch auf Blutverträglichkeit, d.h. möglichst ausgeprägte antithrombogene Eigenschaften an. Nach der internationalen Patentanmeldung WO 94/17904 lassen sich Membranen für medizinische Zwecke durch Behandlung mit einem Niederdruckplasma so modifizieren, daß u.a. ihre thrombogenen Eigenschaften gegenüber unbehandelten Membranen herabgesetzt sind. Unter den geeigneten Plasma-bildenden Gasen wird auch Schwefeldioxid aufgeführt. J.-C. Lin et al. beschreiben in Biomaterials **16** (1995), 1017-1023, die Plasma-Behandlung der inneren Oberfläche von LDPE-Rohren, wobei wiederum Schwefeldioxid als plasmabildendes Gas eingesetzt werden kann. Die Autoren berichten, daß die durch SO₂-Plasma modifizierten Oberflächen Sulfonatgruppen enthielten und stark hydrophil, aber in höherem Maße thrombogen waren als die unbehandelten Oberflächen. Die Autoren vermuten, daß dies auf die kombinierte Wirkung von Oberflächenchemie, also der Sulfonierung, und der Hydrophilität der Oberflächen zurückgeht.

### 2. Kurzbeschreibung der Erfindung

Der vorliegenden Erfindung lag die Aufgabe zugrunde, ein nachhaltig blutverträgliches und bakterienabweisendes Polymer bereitzustellen, das ohne Wirkungsverlust auf der Oberfläche von Polymersubstraten durch kovalente chemische Bindungen und daher dauerhaft fixiert werden kann und keine Inaktivierung durch abgetötete Bakterien oder sonstige Ablagerungen zeigt.

Es wurde überraschenderweise gefunden, daß diese Aufgabe gelöst wird durch ein Copolymer, das (a) mindestens ein sulfonsäure- oder sulfonatgruppenhaltiges Monomer, (b) mindestens ein carboxylgruppen- oder carboxylatgruppenhaltiges Monomer und (c) mindestens ein Monomer mit einer NCO-reaktiven Gruppe enthält

Ein anderer Gegenstand der Erfindung ist ein Verfahren zur Herstellung des genannten Copolymers durch radikalisch initiierte Polymerisation der genannten Monomeren.

Bei einer besonderen Ausführungsform des Verfahrens wird das genannte Copolymer, das aufgrund seines Anteiles an Monomer (c) bereits NCO-reaktiv ist, in einer polymeranalogen Reaktion mit einer polyfunktionellen NCO-reaktiven Verbindung umgesetzt, wodurch weitere NCO-reaktive Gruppen eingeführt werden.

Ein weiterer Gegenstand der Erfindung ist das nach dieser besonderen Ausführungsform des Verfahrens erhältliche Copolymer mit den weiteren NCO-reaktiven Gruppen.

Ebenfalls ein Gegenstand der Erfindung ist ein Verfahren zur Modifizierung von hydrophilen Oberflächen von Polymersubstraten, bei dem ein erfindungsgemäßes Copolymer, das die erwähnt mit einer polyfunktionellen NCO-reaktiven Verbindung umgesetzt worden sein kann, durch Umsetzung mit einem Polyisocyanat kovalent auf der Oberfläche gebunden wird.

Schließlich sind Gegenstände der Erfindung Erzeugnisse mit derart modifizierten Oberflächen sowie deren Verwendung für hygienische, technische, nahrungsmittel- und biotechnische sowie insbesondere für medizinische Zwecke.

### 3. Vorteile der Erfindung

Das erfindungsgemäße Copolymer vermindert die Adhäsion und Vermehrung von Bakterien in einem hohen Maße auch über lange Zeit. Diese Wirkung wird durch die Fixierung auf einem Polymersubstrat über kovalente Bindungen nicht beeinträchtigt. Von dieser Wirkung betroffene Bakterien sind u.a. Staphylococcus aureus, Staphylococcus epidermidis, Streptococcus pyogenes, Klebsiella pneumoniae, Pseudomonas aeruginosa und Escherichia coli. Wenn es insbesondere bei medizinischen Verwendungen darauf ankommt, daß das Copolymer frei von migrationsfähigen Monomeren oder Oligomeren ist, kann man diese durch langsames Umfällen entfernen. Dazu wird das Copolymer in Wasser gelöst und die Lösung langsam in Ethanol eingerührt, wodurch das Copolymer gefällt wird. Das fixierte Copolymer kann durch Extraktion Mit Wasser oligomeren- und monomerenfrei erhalten werden. Die physikalischen und chemischen Eigenschaften des Substratmaterials bleiben nach der Oberflächenmodifizierung praktisch unverändert. Unerwünschte Nebenwirkungen durch freigesetzte körperfremde Stoffe oder durch abgetötete Bakterien treten nicht ein.

### 4. Erfindungsgemäße Copolymere und ihre Herstellung

Das erfindungsgemäße Copolymer enthält die Monomeren (a), (b) und (c), die olefinisch ungesättigt und in einer radikalisch initiierten Reaktion zum Copolymer, einem Terpolymer, polymerisierbar sind. Von den geeigneten **Monomeren (a)** seien beispielsweise Vinylsulforsäure, Methallylsulfonsäure, 2-Acrylamido-2-methyl-1-propansulfonsäure und insbesondere 4-Styrolsulfonsäure sowie deren Alkalisalze, insbesondere die Natriumsalze genannt.

Geeignete **Monomere (b)** sind z.B. Acrylsäure, Methacrylsäure, 4-Vinylsalicylsäure, Itaconsäure, Vinylessigsäure, Zimtsäure, 4-Vinylbenzoesäure, 2-Vinylbenzoesäure, Crotonsäure, Isocrotonsäure, Methylmaleinsäure, Dihydroxymaleinsäure, Fumarsäure, Methylfumarsäure, Dimethylfumarsäure, Allylessigsäure und besonders Maleinsäure sowie deren Alkalisalze und wiederum insbesondere die Natriumsalze,

Die für die Polymerisation eingesetzten Monomeren (a) und (b) können anstelle der Sulfonsäure- oder Sulfonatgruppen bzw. Carboxyl- oder Carboxylatgruppen zunächst davon abgeleitete Gruppen enthalten, z.B. Ester- und Amidgruppen sowie, soweit es Carboxylgruppen betrifft. Nitril- oder Anhydridgruppen, die nach der Polymerisation in üblicher Weise hydrolytisch in Sulfosäure- bzw. Carboxylgruppen umgewandelt werden. Weiterhin kann man bei der Polymerisation von sulfonsäuregruppenhaltigen und/oder carboxylgruppenhaltigen Monomeren ausgehen und die sauren Gruppen nachträglich ganz oder teilweise neutralisieren, z.B. mit Natriumhydroxid. Bei teilweiser Neutralisierung liegen saure Gruppen und deren Salze nebeneinander vor.

Zu den geeigneten **Monomeren (c)** zählen insbesondere solche mit Hydroxylgruppen oder primären oder sekundären Aminogruppen. Dazu gehören Hydroxyalkylacrylate und -methacrylate, wie Hydroxyethylacrylat, das besonders bevorzugte Hydroxyethylmethacrylat (HEMA), 4-Hydroxybutylacrylat, 4-Hydroxybutylmethacrylat, 3-Hydroxypropylacrylat und 3-Hydroxypropylmethacrylat; ungesättigte Alkohole oder Phenole, wie Allylalkohol, 2,3-Butadien-1-ol, 2-Buten-1,4-diol, 3-Methylallyalkohol, o-, m- und p-Hydroxystyrol; sowie Dihydroxymaleinsäure, die zugleich ein Monomer (b) ist. Von den geeigneten aminogruppenhaltigen Monomeren (c) sei beispielsweise das 4-Aminostyrol und Diallylamin genannt.

Natürlich kann man anstelle eines einzelnen Monomers (a), (b) oder (c) entsprechende Mischungen einsetzen, z.B. statt Natriumstyrolsulfonat allein eine Mischung aus Natriumstyrolsulfonat und Natriumvinylsulfonat.

In manchen Fällen ist es möglich und zweckmäßig, die biologischen und/oder die Verarbeitungseigenschaften des Copolymers durch bis zu 70 Molprozent, bezogen auf die Summe der Monomeren (a), (b) und (c), von **Monomeren (d)** mit anderer oder ohne Funktionalität zu modifizieren, z.B. um die Löslichkeit in anderen Lösemitteln als Wasser zu erhöhen. Als Beispiele für solche Monomere seien Vinylester, wie Vinylacetat und Vinylpropionat; Vinylketone, wie Vinylmethylketon und Vinylbutylketon; Vinylaromaten, wie Styrol und Vinyltoluol; Olefine, wie 1-Buten, 1-Hexen und 1-Octen; und (Meth)acrylate, wie Methylmethacrylat, Methylacrylat und n-Butylacrylat genannt.

Die Monomeren (a), (b), (c) und gegebenenfalls (d) können im Copolymer, je nach Reaktionsführung, als Blöcke oder statistisch verteilt vorliegen. Von den drei funktionellen Gruppen des Copolymers ergeben die Sulfonsäure- und/oder Sulfonat- und die Carbonsäure- und/oder Carboxylatgruppen zusammen die erwünschten blutverträglichen und bakterienabweisenden Eigenschaften, während die NCO-reaktiven Gruppen die kovalente Fixierung (oder Anbindung) des Copolymers auf der Oberfläche des Substrats ermöglichen.

Die molaren Verhältnisse der drei funktionellen Gruppen können in weiten Grenzen schwanken. Vorteilhaft liegen Carboxyl- und/oder Carboxylatgruppen sowie Sulfonsäure- und/oder Sulfonatgruppen im Molverhältnis von 0,1 bis 10, vorteilhaft von 0,2 bis 10 und insbesondere von 0,2 bis 5 vor. Die NCO-reaktiven Gruppen sind zweckmäßig in Mengen von 5 bis 30, insbesondere von 5 bis 20 Mol-%, Molprozent vorhanden, bezogen auf die Summe von Carboxyl-, Carboxylat-, Sulfonsäure-, Sulfonat und NCO-funktionellen Gruppen.

Die Polymeren werden in üblicher Weise durch radikalisch initiierte Polymerisation der Monomeren (a), (b), (c) und gegebenenfalls (d) hergestellt, vorteilhaft durch Lösungs- oder Emulsionspolymerisation. Ein besonders gut geeignetes Lösemittel ist Wasser, in dem die Monomeren und auch das Copolymer in der Regel gut löslich sind. Bei geringen Gehalten an dem Monomer (c) sind die Monomeren und ist das Copolymer auch in stark polaren organischen Lösemitteln, wie Dimethylformamid, Dimethylacetamid und Dimethylsulfoxid, zumindest teileweise löslich und im übrigen emulgierbar.

Geeignete Polymerisationsinitiatoren sind z.B. Azonitrile, Alkylperoxide, Acylperoxide, Hydroperoxide, Peroxodisulfate, Peroxyketone, Peroxyester und Percarbonate sowie alle üblichen Photoinitiatoren. Man wendet sie in der Regel in Mengen von 0,01 bis 1,5 Mol-% an, bezogen auf die Summe der Monomeren.

Die Polymerisation wird thermisch, z.B. durch Erhitzen auf 60 bis 100°C, oder durch Strahlung mit entsprechender Wellenlänge initiiert. Man kann z.B. einen Teil der Monomeren (a), (b), (c) und gegebenenfalls (d) vorlegen, die Polymerisation starten und die restlichen Monomeren als Gemisch in den Reaktor einführen, wobei man durch Kühlen zweckmäßig eine Temperatur von 60 bis 90°C aufrechterhält. Auf diese Weise erhält man ein statistisches Copolymer. Wenn man ein bestimmtes Monomer oder einen Anteil davon vorlegt und die anderen Monomeren und gegebenenfalls die Restmenge des vorgelegten Monomers jeweils für sich portionsweise zugibt, entsteht ein Block-Copolymer. Natürlich sind diese Varianten idealtypische Grenzfälle. In der Praxis erhält man schon infolge der unterschiedlichen Reaktionsgeschwindigkeit der Monomeren im ersten Fall neben Bereichen mit statistischer Verteilung auch solche mit überwiegender Blockstruktur und im zweiten Falle neben den Blöcken auch Übergangszonen mit mehr oder minder gleichmäßiger statistischer Verteilung der Bausteine. Auf die Bioaktivität des Copolymers hat die Verteilung der Monomeren in der Kette keinen merklichen Einfluß.

Nach Beendigung der Polymerisation erhält man, je nach den Anteilen der Monomeren (a), (b), (c) und gegebenenfalls (d) und dem verwendeten Lösemittel, eine Lösung oder Emulsion des Copolymers mit Feststoffgehalten, die z.B. 40 bis 60 Gew.-% betragen können. Diese Lösung oder Emulsion kann direkt zur Modifizierung von hydrophilen Oberflächen, insbesondere von hydrophilen Kunststoffoberflächen verwendet werden. Wenn das Copolymer isoliert soll, kann man z.B. die Lösung in ein Lösemittel gießen, in dem das Copolymer unlöslich ist, z.B. in Ethanol, und das Copolymer in üblicher Weise abtrennen.

### 5. Umsetzung der erfindungsgemäßen Copolymeren mit polyfunktionellen NCO-reaktiven Verbindungen

Wie zuvor erwähnt, wird bei einer besonderen Ausführungsform des Verfahrens das erfindungsgemäße Copolymer, das aufgrund seines Anteiles an Monomer (c) bereits NCO-reaktiv ist, in einer polymeranalogen Reaktion mit einer polyfunktionellen NCO-reaktiven Verbindung umgesetzt, wodurch weitere NCO-reaktive Gruppen eingeführt werden, insbesondere Hydroxyl- oder primäre bzw. sekundäre Aminogruppen. Auf diese Weise wird die Verknüpfungsdichte bei der nachfolgenden kovalenten Fixierung des Copolymers auf einem hydrophilen Polymersubstrat erhöht, Für die Umsetzung eignen sich vor allem Copolymere mit Carboxyl- und Sulfonatgruppen. Nach der Umsetzung können durch Behandlung mit Säure Sulfonatgruppen in Sulfosäuregruppen bzw. mit Basen Carboxylgruppen in Carboxylatgruppen umgewandelt werden, wenn dies erwünscht ist.

Als polyfunktionelle NCO-reaktive Verbindungen eignen sich vorzugsweise Polyole, wie Diole oder Triole; Aminoalkohole, wie Amino- oder N-Alkylaminoalkanole; sowie Polyamine, wie Diamine und Triamine. Als Beispiele seinen genannt: Ethylenglykol, Propylenglykol, 1,4-Butandiol, 1.6-Hexandiol, Trimethylolpropan, Aminoethanol, N-Methylaminoethanol, Ethylendiamin, Propylendiamin, Hexamethylendiamin und Diethylentriamin. Die NCO-reaktiven Gruppen sind zugleich Carboxylreaktiv und reagieren bei der Umsetzung mit Carboxylgruppen des erfindungsgemäßen Copolymers unter Bildung von Estergruppen bzw. Carbonamidgruppen und unter Einführung weiterer NCO-reaktiver Gruppen.

Da bei der Modifizierung des erfindungsgemäßen Copolymers durch Umsetzung mit der polyfunktionellen NCO-reaktiven Verbindung Carboxylgruppen verbraucht werden, muß der molare Anteil der Carboxylgruppen an der Gesamtfunktionalität des unmodifizierten Copolymers entsprechend höher sein, wenn ein bestimmtes Verhältnis von Carboxyl- und Carboxylatgruppen zu Sulfonsäure- und Sulfonatgruppen und ein bestimmter molarer Anteil an NCO-reaktiven Gruppen an der Gesamtfunktionalität des modifizierten Copolymers vorgegeben ist. Die Menge der bei dieser Umsetzung eingesetzten polyfunktionellen NCO-reaktiven Verbindung hängt von deren Funktionalität (bi-, trifunktionell), dem molaren Anteil der Carboxylgruppen im unmodifizierten Copolymer und dem gewünschten molaren Verhältnis von Carboxyl- und Carboxylatgruppen zu Sulfonsäure- und Sulfonatgruppen im modifizierten Copolymer ab. Der Anteil der NCO-reaktiven Gruppen an der Gesamtfunktionalität des modifizierten Copolymers (d.h. Sulfonat-, Carboxyl- und NCO-reaktive Gruppen) liegt höher als beim unmodifizierten Copolymer und beträgt zweckmäßig etwa 10 bis 30, insbesondere etwa 10 bis 50 Molprozent.

Zur NCO-reaktiven Modifizierung kann das erfindungsgemäße Copolymer in einem Autoklaven mit einem Aminoalkohol, z.B. Ethanolamin, oder einem Polyamin, z.B. Hexamethylendiamin, bei erhöhter Temperatur und erhöhtem Druck umgesetzt werden. Der Verlauf der Umsetzung kann mittels potentiometrischer Titration oder NMR-Spektroskopie verfolgt werden. Alternativ kann die Modifizierung auch drucklos vorgenommen werden, indem man das primäre Copolymer und ein Polyol, z.B. ein Polyethylenglykol, einem hochsiedenden Lösemittel erhitzt, zweckmäßig in Gegenwart eines Schleppmittels für Wasser.

### 6. Modifizierung von hydrophilen Oberflächen mit den Copolymeren gemäß 4. und 5.

Ebenfalls ein Gegenstand der Erfindung ist ein Verfahren zur Modifizierung von hydrophilen Oberflächen von Polymersubstraten, bei dem ein erfindungsgemäßes Copolymer, mit oder ohne nachträgliche Modifizierung durch Umsetzung mit einer polyfunktionellen NCO-reaktiven Verbindung, durch Umsetzung mit einem Polyisocyanat kovalent auf der Oberfläche gebunden wird.

Dabei wird das Copolymer durch kovalente Bindungen auf der Oberfläche des hydrophilen Substrats fixiert. Das Polyisocyanat reagiert mit den NCO-reaktiven Gruppen des Copolymers und mit Hydroxylgruppen, die auf der Oberfläche des Substrats vorhanden sind, unter Ausbildung von Urethanbindungen. Aus Aminogruppen auf der Oberfläche des Substrats entstehen in entsprechender Weise Harnstoffbrücken. Natürlich reagiert ein Teil der Polyisocyanat-Moleküle ausschließlich mit Hydroxylgruppen des Copolymers unter Kettenverlängerung oder -verknüpfung bzw. -vernetzung. Trotzdem erreicht man bei den genannten molaren Anteilen von 5 bis 50 Mol-% an NCO-reaktiven Gruppen eine dauerhafte und feste Bindung des Copolymers an das Polymersubstrat.

Wenn das Copolymer in wäßriger Lösung oder Emulsion oder in einem anderen, mit NCO-Gruppen reaktiven Medium vorliegt, müssen die Isocyanatgruppen des Polyisocyanats zunächst geschützt (oder maskiert) werden. Dies geschieht in üblicher Weise mit Verbindungen, die sich bei niedrigen Temperaturen, wie 20 bis 60°C, an die NCO-Gruppen anlagern und bei höheren Temperaturen wieder abspalten. Solche Verbindungen sind z.B. Methylethylketoxim, Acetonoxim, ε-Caprolactam, Ethylacetoacetat, Malonsäureester, Glykolsäurebutylester, Diisopropylamin, 3,5-Dimethylpyrazol und 1,2,4-Triazin. Ein gut geeignetes Polyisocyanat zur Verwendung in wäßrigen Systemen ist beispielsweise mit Methylethylketoxim blockiertes Isophorondiisocyanat (IPDI). Fällt das Copolymer in organischen, gegenüber NCO-Gruppen inerten Lösemitteln gelöst oder emulgiert an, z.B. in Dimethylformamid, so kann man mit einem unblockierten Polyisocyanat arbeiten, z.B. wiederum mit IPDI oder mit Hexamethylendiisocyanat (HDI), 4,4'-Methylendi(phenylisocyanat) (MDI), dessen Hydrierungsprodukt (H-MDI) oder Toluoldiisocyanat (TDI).

Überraschenderweise hat sich herausgestellt, daß die Mitverwendung eines Kettenverlängerungsmittels bei der Anbindung eines erfindungsgemäßen NCO-reaktiven Copolymers zu einer - mittels ESCA nachweisbaren - höheren Konzentratrion an bioaktiven Gruppen auf dem Substrat und damit zu einer verstärkten Wirkung führt. Kettenverlängerungsmittel sind Verbindungen mit in der Regel endständigen NCOreaktiven Gruppen, wie Hydroxyl- oder Aminogruppen. Gut geeignet sind z.B. Diamine, Aminoalkohole und Diole, insbesondere Polyalkylenglykole, mit (gegebenenfalls mittleren) Molgewichten von etwa 100 bis 3.000 in Mengen beispielsweise von 10 bis 100 Gew.-%, bezogen auf das Copolymer.

### 7. Modifizierbare Polymersubstrate

Die Polymeren, deren Oberfläche erfindungsgemäß modifiziert werden, können Homo- oder Copolymere sein, die ihrer Natur nach hydrophil oder hydrophiliert sind, wie in der Folge erläutert, d.h. auf der Oberfläche Hydroxylgruppen und/oder Aminogruppen tragen. Hydrophile und hydrophilierte (Co)polymere werden hier gemeinsam als hydrophil bezeichnet. Zu den im Prinzip geeigneten Basis(co)polymeren zählen z.B. Polyolefine, wie Polyethylen, Polypropylen, Polyiscobutylen, Polybutadien, Polyisopren, natürliche Kautschuke und Polyethylen-copropylen; halogenhaltige Polymere, wie Polyvinylchlorid, Polyvinylidenchlorid, Polychloropren, Polytetrafluorethylen und Polyvinylidenfluorid; Polymere und Copolymere aus vinylaromatischen Monomeren, wie Polystyrol, Polyvinyltoluol, Polystyrol-co-vinyltoluol, Polystyrol-co-acrylnitril, Polystyrol-co-butadien-co-acrylnitril; Polykondensate, beispielsweise Polyester, wie Polyethylenterephthalat und Polybutylenterephthalat; Polyamide, wie Polycaprolactam, Polylaurinlactam und das Polykondensat aus Adipinsäure und Hexamethylendiamin; Polyetherblockamide, z.B. aus Laurinlactam oder Caprolactam und Polyethylenglykol mit durchschnittlich 8, 12 oder 16 Ethoxygruppen; weiterhin Polyurethane, Polyether, Polycarbonate, Polysulfone, Polyetherketone, Polyesteramide und imide, Polyacrylnitril, Polyacrylate und -methacrylate.

Soweit die Polymeren oder Copolymeren nicht hinreichend hydrophil sind, müssen sie hydrophiliert werden. Das ist dann der Fall wenn der Kontaktwinkel von Wasser bei 25°C, gemessen nach dem Verfahren von R.J.Good et al., Techniques of Measuring Contact Angles in Surface and Colloid Sciences, Vol. 11, Plenum Press New York, N.Y., 1979, >36° beträgt. Für die Hydrophilierung steht eine ganze Reihe von Methoden zur Verfügung. So kann man hydroxylgruppenhaltige Monomere, wie Hydroxyethyl(meth)acrylat oder Hydroxybutyl(meth)acrylat, strahleninduziert auf eine polymere Substratoberfläche aufpfropfen (deutsche Patentanmeldung 197 00 079.7 (O.Z. 5146)). Statt Monomere zu verwenden, kann man auch ein hydroxylgruppenhaltiges Copolymer auf die Substratoberfläche pfropfen (deutsche Patentanmeldung 197 00 081.9). Weiterhin kann man derartige Copolymere in üblicher Weise auf die Substratoberfläche aufbringen. z.B. durch Spritzen, Tauchen oder Spin-Coating mit Lösungen der Copolymeren. Weiterhin lassen sich nicht hinreichend hydrophile Polymere oder Copolymere durch Behandlung mit Argon-Plasma oder Bestrahlung mit UV-Strahlen von 100 bis 400 nm hydrophilieren. Andere bekannte Hydrophilierungsmethoden sind Beflammen, Corona- und Ozonbehandlung. Durch Behandlung mit Ammoniak-Plasma erreicht man nicht nur eine Hydrophilierung, sondern durch Einführung von Aminogruppen zusätzliche Bindungsmöglichkeiten sowie physiologische Effekte. Schließlich führt auch eine Ätzung mit starken Säuren, wie Schwefelsäure, Salzsäure und Salpetersäure, oder Basen, wie Alkalimetallhydroxiden, zu einer hinreichenden Hydrophilierung von hydrophoben Polymeren oder Copolymeren.

Die hydrophilen oder hydrophilierten Substratoberflächen werden mit der Lösung oder Emulsion des Copolymers, die ein gegebenenfalls blockiertes Polyisocyanat enthält, in üblicher Weise beschichtet, z.B. durch Tauchen, Spritzen oder Spin-Coating. Nach dem Verdampfen des Lösemittels und gegebenenfalls nach Abspaltung des Blockierungsmittels findet bei 120 bis 200°C, insbesondere 160 bis 190°C (?) die Fixierung des Copolymers auf der Substratoberfläche statt. Man hält diese Temperatur zweckmäßig 5 Sekunden bis 30 Minuten, vorteilhaft 10 Sekunden bis 5 Minuten aufrecht und erhält dann eine kovalent gebundene, sehr abriebbeständige Beschichtung.

Bei einer Variante des Beschichtungsverfahrens behandelt man zunächst die hydrophile oder hydrophilierte Substratoberfläche mit dem gegebenenfalls blockierten Polyisocyanat, um das Polyisocyanat mittels einer seiner NCO-Gruppen auf der Oberfläche zu fixieren, und bringt dann auf die vorbehandelte Fläche die Lösung oder Emulsion des Copolymers auf, dessen NCO-reaktiven Gruppen mit den verbliebenen NCO-Gruppen des Polyisocyanats reagieren.

### 7. Erfindungsgemäße Erzeugnisse

Erzeugnisse mit erfindungsgemäß modifizierten Oberflächen eignen sich für viele Verwendungen, bei denen es darauf ankommt. Bakterienadhäsion und -wachstum zu vermeiden oder zurückzudrängen. Sie sind also u.a. für hygienische, technische, nahrungsmittel- und und biotechnische Zwecke geeignet, wie sie beispielhaft zuvor beschrieben wurden. Die Gegenstände eignen sich vorzugsweise für medizinische Verwendungen, besonders dann, wenn es gleichzeitig auf bakterienabweisende Eigenschaften und Blutverträglichkeit ankommt. Aber auch bei Kontakten mit anderen Körperflüssigkeiten als Blut, wie Lymphe, oder mit Gewebe kommen die vorteilhaften Eigenschaften der erfindungsgemäßen Gegenstände zur Geltung. Medizinische Verwendungen sind z.B. solche als Katheter, Lymphdrainagen, Wundverbände, Tubusse, Stents, Herzklappen oder als Schläuche bei der Peritonealdialyse.

Die folgenden Beispiele sollen die Erfindung weiter erlautern, nicht aber ihren Umfang begrenzen, wie er in den Patentansprüchen dargelegt ist.

### Beispiele

Die folgenden Monomeren wurden eingesetzt:

**Tabelle 1**

| **SO**_{**3**}^{**-**}**-Monomere** | |
|---|---|
| Monomer | Abkürzung |
| Natrium-4-styrolfulfonat | S1 |
| Natriummethallylsulfonat | S2 |
| Natriumvinylsulfonat | S3 |

**Tabelle 2**

| **COOH-Monomere** | |
|---|---|
| Monomer | Abkürzung |
| Acrylsäure | C1 |
| Maleinsäure | C2 |
| Fumarsäure | C3 |
| Methacrylsäure | C4 |

**Tabelle 3**

| **OH-Monomere** | |
|---|---|
| Monomer | Abkürzung |
| Hydroxyethylmethacrylat | H1 |
| Polyethylenglykol-1000-monomethylether-monomethacrylat | H2 |
| Glycidylmethacrylat | H3 |
| Hydroxyethylacrylat | H4 |

Die in Tabelle 4 aufgeführten erfindungsgemäßen Copolymeren wurden durch Lösungspolymerisation unter den dort angegebenen Bedingungen hergestellt. Mittels Dünnschichtchromatographie wurde geprüft, ob bzw. wie weit die Polymerisation erfolgt war. Die Polymeren wurden durch Einrühren des Polymerisationsgemisches in Ethanol ausgefällt, mit Ethanol gewaschen und getrocknet.

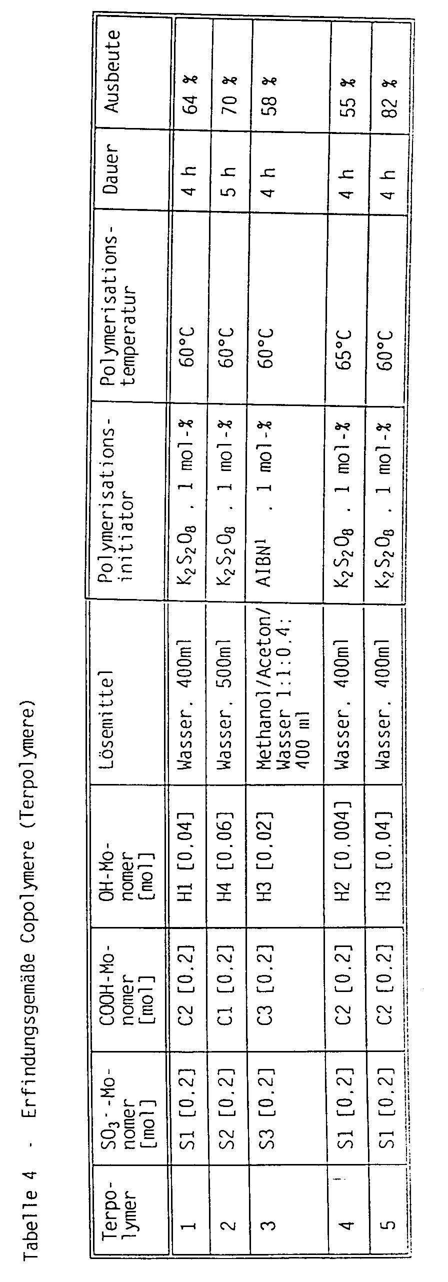

### Bestimmung der primären Bakterienadhäsion unter statischen Bedingungen

Eine Über-Nacht-Kultur des Bakterienstammes Klebsiella pneumoniae in Hefeextrakt-Pepton-Glukose-Nährmedium (1% + 1% + 1%) wird abzentrifugiert und in Phosphat-gepufferter Saline (=PBS; 0,05m KH₂PO₄, pH 7,2 + 0,9 % NaCl) wieder aufgenommen. Man verdünnt mit PBS-Puffer auf ein Zellkonzentration von 10⁸ Zellen/ml. Die suspendierten Bakterien werden mit dem zu untersuchenden Folienstück für 3h in Berührung gebracht. Dazu werden doppelseitig beschichtete kreisförmige Folienstücke mit einem Durchmesser von 1,6 cm (=4,02 cm²) auf eine Präpariernadel gesteckt und mit der Zellsuspension geschüttelt. Einseitig beschichtete Folien werden in Form einer runden, ebenen Scheibe von 4,5 cm Durchmesser und mit einer Stützmembran aus 2-3 cm dickem Weich-PVC in eine Membranfilterapparatur eingespannt. Auf die nach oben zeigende Seite mit der zu prüfenden Beschichtung wird die Zellsuspension aufgegeben und 3h geschüttelt. Die Membranfilterapparatur muß dicht sein, d.h. es darf keine Zellsuspension durch undichte Zellen ausfließen.

Nach Ablauf der Kontaktzeit wird die Bakteriensuspension mit einer Wasserstrahlpumpe abgesaugt, und die Folienstücke werden zum Waschen mit 20 ml steriler PBS-Lösung in einem 100 ml Becherglas 2 min geschüttelt. Das Folienstück wird nochmals in sterile PBS-Lösung eingetaucht und dann in 10 ml erhitztem TRIS/EDTA (0,1M Trishydroxyethylaminomethan, 4 mM Ethylendiamintetraessigsäure, mit HCl auf pH 7,8 eingestellt) für 2 min im siedenden Wasserbad extrahiert.

Mit der Extraktionslösung werden kleine Eppendorf-Cups befüllt und sofort bis zur Biolumineszenz-Bestimmung des extrahierten Adenosintriphosphats (ATP) bei -20°C eingefroren. Die Bestimmung wird wie folgt ausgeführt: In ein transparentes Röhrchen aus Polycarbonat wird 100 µl Reagentienmix (Biolumineszenz-Test CLS II, Fa. BOEHRINGER MANNHEIM GmbH) gegeben, und über einen Zeitraum von 10 sec werden in einem Lichtimpuls-Meßgerät LUMAT LB9501 (Laboratorien Prof. Berthold GmbH, 75323 Bad Wildbad, Deutschland) die Lichtimpulse integriert. Dann wird eine 100 µl Probe zugegeben und erneut gemessen. Die relativen Lichteinheiten (RLU) werden durch Subtraktion der Lichtimpulse im Reagentienmix von der Anzahl der gemessenen Lichtimpulse im kompletten Ansatz erhalten. Dieser Wert steht in Relation zu der Anzahl der an der Folie adhärierten Bakterien. Der Umrechnungsfaktor zwischen dem RLU-Wert und der Bakterienzahl wird bestimmt, indem ein Aliquot von 0,1 ml der Bakteriensuspension mit 10⁸ Zellen/ml in 10 ml heißem TRIS/EDTA extrahiert und dann der ATP-Gehalt bestimmt wird.

In der folgenden Tabelle 5 sind die Bedingungen für die Beschichtung der verschiedenen Polymersubstrate mit den erfindungsgemäßen Copolymeren sowie die Elementenzusammensetzung auf der modifizierten Oberfläche und die Ergebnisse der Messungen der primären Bakterienadsorption zusammengestellt.

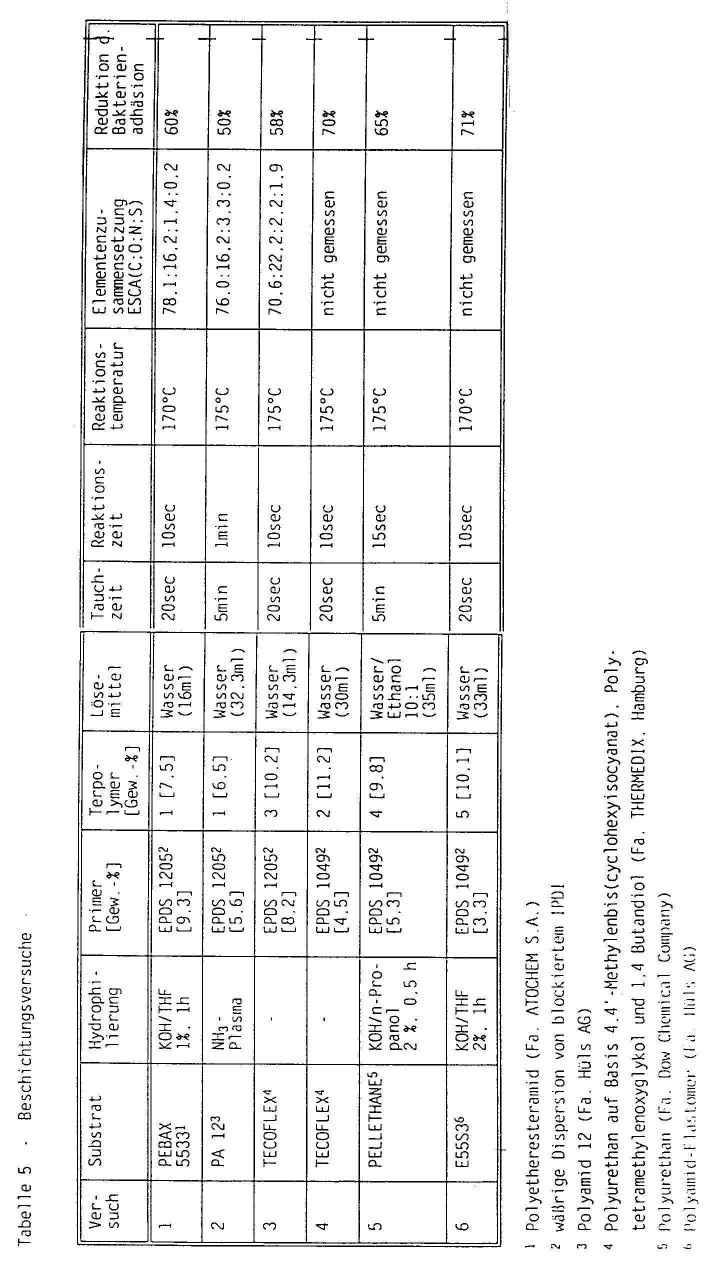

## Patentansprüche

1. Copolymer, enthaltend (a) mindestens ein sulfonsäure- oder sulfonatgruppenhaltiges Monomer, (b) mindestens ein carboxyl- oder carboxylatgruppenhaltiges Monomer und (c) mindestens ein Monomer mit einer NCO-reaktiven Gruppe.

2. Copolymer nach Anspruch 1, dadurch gekennzeichnet, daß bis zu 70 Molprozent, bezogen auf die Summe der Monomeren (a), (b) und (c). Monomere mit anderer Funktionalität als derjenigen der genannten Monomeren oder ohne Funktionalität enthalten sind.

3. Copolymer nach Anspruch 1 oder 2, dadurch gekennzeichnet, daß das sulfonatgruppenhaltige Monomer (a) Natrium-p-styrolsulfonat ist.

4. Copolymer nach einem der Ansprüche 1 bis 3, dadurch gekennzeichnet, daß das carboxylgruppenhaltige Monomer (b) Maleinsäure ist.

5. Copolymer nach einem der Ansprüche 1 bis 4, dadurch gekennzeichnet, daß das Monomer mit NCO-reaktiver Gruppe (c) Hydroxyethylmethacrylat ist.

6. Copolymer nach einem der Ansprüche 1 bis 5, dadurch gekennzeichnet, daß das Molverhältnis der Carboxyl - und/oder Carboxylatgruppen zu den Sulfosäure- und/oder Sulfonatgruppen 0,1 bis 10 beträgt.

7. Copolymer nach einem der Ansprüche 1 bis 6, dadurch gekennzeichnet, daß es zusätzliche, durch Umsetzung mit einer polyfunktionellen NCO-reaktiven Verbindung eingeführte NCO-reaktive Gruppen aufweist.

8. Copolymer nach einem der Ansprüche 1 bis 7, dadurch gekennzeichnet, daß der molare Anteil der NCO-reaktiven Gruppen, bezogen auf die Summe der molaren Anteile der Sulfonsäure-, Sulfonat-, Carboxyl-, Carboxylat- und NCO-reaktiven Gruppen, 5 bis 50 % beträgt.

9. Verfahren zur Herstellung eines Copolymers aus (a) mindestens einem sulfonsäure- oder sulfonatgruppenhaltigen Monomer, (b) mindestens einem carboxyl- oder carboxylatgruppenhaltigen Monomer und (c) mindestens einem Monomer mit einer NCO-reaktiven Gruppe, dadurch gekennzeichnet, daß man die Monomeren radikalisch initiiert polymerisiert.

10. Verfahren nach Anspruch 9, dadurch gekennzeichnet, daß die Polymerisation eine Lösungspolymerisation mit Wasser als Lösemittel ist.

11. Verfahren zur Modifizierung von hydrophilen Oberflächen von Polymersubstraten, dadurch gekennzeichnet, daß ein Copolymer nach einem der Ansprüche 1 bis 7 durch Umsetzung mit einem Polyisocyanat kovalent auf der Oberfläche gebunden wird.

12. Verfahren nach Anspruch 10, dadurch gekennzeichnet, daß der molare Anteil der NCO-reaktiven Gruppen, bezogen auf die Summe der molaren Anteile der Sulfonsäure-, Sulfonat-, Carboxyl-, Carboxylat- und NCO-reaktiven Gruppen, 5 bis 50 % beträgt.

13. Verfahren nach Anspruch 11 oder 12, dadurch gekennzeichnet, daß die Umsetzung in wäßrigem Medium erfolgt und das Polyisocyanat ein verkapptes Polyisocyanat ist.

14. Erzeugnisse mit einer nach einem Verfahren gemäß Anspruch 11 bis 13 modifizierten Oberfläche.

15. Erzeugnisse nach Anspruch 14, dadurch gekennzeichnet, daß sie Katheter, Schläuche, Membranen, Blutbeutel, Wundverbände, Tubusse, Stents oder Herzklappen sind.

16. Verwendung von Erzeugnissen nach Anspruch 14 für hygienische, technische, nahrungsmittel- oder biotechnische Zwecke.

17. Verwendung von Erzeugnissen nach Anspruch Anspruch 14 oder 15 für medizinische Zwecke.
